(19) Europäisches Patentamt
European Patent Office
Office européen des brevets

(11) EP 0 626 372 B1

(12) FASCICULE DE BREVET EUROPEEN

(45) Date de publication et mention de la délivrance du brevet: **20.12.2000 Bulletin 2000/51**

(51) Int. Cl.[7]: **C07D 233/54**, A61K 31/415

(21) Numéro de dépôt: **94870084.4**

(22) Date de dépôt: **19.05.1994**

(54) **2-Hydroxy-3-[1-(1H-imidazol-4-yl)alkyl]-benzènecarboximidamides pour le traitement du glaucome**

2-Hydroxy-3-[1-(1H-imidazol-4-yl)Alkyl]-Benzol Carboximid-Amide zur Glaucoma-Behandlung

2-hydroxy-3-[1-(1H-imidazol-4-yl)alkyl]benzenecarboximid-amides for the treatment of glaucoma

(84) Etats contractants désignés:
**AT BE CH DE DK ES FR GB GR IE IT LI LU MC NL PT SE**
Etats d'extension désignés:
**SI**

(30) Priorité: **27.05.1993 GB 9310965**

(43) Date de publication de la demande:
**30.11.1994 Bulletin 1994/48**

(73) Titulaire: **UCB, S.A.**
**1070 Bruxelles (BE)**

(72) Inventeurs:
- **Michel, Philippe**
  **B-1650 Beersel (BE)**
- **Cossement, Eric**
  **B-1050 Bruxelles (BE)**
- **Gobert, Jean**
  **B-1040 Bruxelles (BE)**
- **Wülfert, Ernst**
  **B-1180 Bruxelles (BE)**

(74) Mandataire:
**Lechien, Monique et al**
**UCB, S.A.,**
**Allée de la Recherche 60**
**1070 Bruxelles (BE)**

(56) Documents cités:
**EP-A- 0 269 599** **EP-A- 0 341 231**
**WO-A-92/09583**

- **JOURNAL OF MEDICINAL CHEMISTRY. vol. 34, no. 11 , 1991 , WASHINGTON US pages 3197 - 3204 D. HUBER ET. AL. 'Synthesis of Ocular Antihypertensive Activity of New Imidazoline Derivatives Containing a .beta.-Blocking Side Chain.'**
- **CHEMICAL ABSTRACTS, vol. 120, no. 23, 1994, Columbus, Ohio, US; abstract no. 290600, NOYER, MICHEL; DE LAVELEYE, FRANCOISE; VAUQUELIN, GEORGES; GOBERT, JEAN; WUELFERT, ERNST 'Mivazerol, a novel compound with high specificity for .alpha.2 adrenergic receptors: binding studies of different human and rat membrane preparations' page 24 ;colonne 3 ; & NEUROCHEM. INT. vol. 24, no. 3 , 1994 pages 221 - 229**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention se rapporte à de nouveaux 2-hydroxy-3-[1-(1H-imidazol-4-yl)alkyl]-benzènecarboximidamides substitués, à leurs sels d'addition d'acides non toxiques ophtalmologiquement acceptables, à des procédés pour les préparer et à leur utilisation dans la prévention et le traitement du glaucome.

Elle concerne également les compositions ophtalmiques renfermant lesdits composés.

Dans la demande de brevet européen n° 269.599 au nom de la demanderesse, on décrit des 1H-imidazoles substitués dont les plus représentatifs sont des 2-hydroxy-3-[1-(1H-imidazol-4-yl)alkyl]-benzèneméthanols. Ces 1H-imidazoles possèdent des propriétés anti-ischémiques cardiaque, cérébrale et tissulaire.

Dans le brevet européen n° 341.231, également au nom de la demanderesse, on décrit des [1-(1H-imidazol-4-yl)alkyl]-benzamides substitués, qui présentent non seulement des propriétés anti-ischémiques cardiaque, cérébrale et tissulaire, mais aussi des propriétés agonistes des récepteurs $\alpha_2$-adrénergiques. Ces dernières propriétés confèrent à ces composés une utilité thérapeutique bénéfique dans le traitement des pathologies induisant ou résultant d'une élévation anormale des taux de catécholamines, telles que, par exemple, la congestion cardiaque, la maladie de Raynaud ou les spasmes des artères coronaires. Pour la même raison, ces composés trouvent aussi une application dans le traitement des troubles associés aux hypersécrétions gastriques et intestinales ainsi que dans le traitement du syndrome de sevrage des toxicomanes. Ces composés présentent en outre une certaine activité diurétique.

Poursuivant ses travaux de recherche dans le domaine, la demanderesse a maintenant synthétisé de nouveaux 2-hydroxy-3-[1-(1H-imidazol-4-yl)alkyl]-benzènecarboximidamides substitués qui sont des agonistes puissants des récepteurs $\alpha_2$-adrénergiques, tout en ne présentant que très peu, voire même pas d'effets secondaires systémiques d'origine centrale ou périphérique. C'est pourquoi, ces composés peuvent avantageusement être utilisés pour réduire la pression intraoculaire et plus particulièrement pour la prévention et le traitement du glaucome.

Le glaucome est une affection oculaire caractérisée par une augmentation de la tension intraoculaire, déterminant un durcissement du globe oculaire, une atrophie du nerf optique avec excavation caractéristique de la papille, un rétrécissement du champ visuel et une diminution plus ou moins marquée de l'acuité visuelle. Le stade terminal du glaucome (ou glaucome absolu) s'accompagne d'une cécité complète du patient.

Le traitement d'urgence du glaucome consiste habituellement en l'application topique d'agents cholinergiques tels que la pilocarpine, d'agents $\alpha$- ou $\beta$-adrénergiques agonistes ou antagonistes tels que la clonidine, le timolol ou l'épinéphrine, ou encore d'inhibiteurs de l'anhydrase carbonique par voie systémique. Enfin, en dernier recours, il est parfois nécessaire de pratiquer une intervention chirurgicale.

Toutefois, les divers traitements traditionnels du glaucome actuellement disponibles s'accompagnent d'effets secondaires dont la nature et la gravité sont très variables.

Ainsi, l'instillation dans l'oeil d'un agent cholinergique tel que la pilocarpine peut provoquer chez certains patients des nausées, de la diarrhée, des spasmes musculaires, de la transpiration, des larmes, de la salivation, etc... Au niveau même de l'oeil, on peut encore observer la contraction de la pupille (myosis) et du muscle ciliaire, de même que la dilatation des vaisseaux sanguins de l'iris et de la conjonctive. Il s'ensuit bien souvent des complications visuelles telles que le spasme d'accommodation, la myopie ou la diminution de l'acuité visuelle.

Le traitement par un agent sympathomimétique tel que la dipivalylépinéphrine est connu pour provoquer fréquemment des sensations de brûlures ou d'irritation. En outre, un effet secondaire important de ces agents est l'apparition de troubles cardiaques tels que palpitations, tachycardie, arythmie, etc...

La clonidine, connue pour être un agoniste des récepteurs $\alpha_2$-adrénergiques, occasionne de la mydriase, ainsi qu'une phase initiale d'hypertension oculaire (effet biphasique). En outre, malgré l'application topique du produit, on observe des effets systémiques importants, tels que de la bradycardie et de l'hypotension.

L'utilisation de médicaments $\beta$-bloquants entraîne également des effets systémiques importants dûs à l'absence de "first-pass effect" lors de l'administration oculaire de ceux-ci. Le timolol, par exemple, provoque de la bradycardie ou de l'hypotension. Ces réactions secondaires systémiques aux médicaments $\beta$-bloquants peuvent atteindre un degré de sévérité tel que l'arrêt du traitement devienne obligatoire. Des cas de dépressions suicidaires, d'hallucinations, de cauchemars ou de psychoses nécessitant une hospitalisation ont été signalés en association avec ces médicaments. De plus, ces composés doivent être utilisés avec infiniment de précautions chez les patients sujets à des troubles fonctionnels cardiaques ou pulmonaires. Chez ce type de patients, entre autres, des cas d'arythmie, d'arrêt cardiaque, d'asthme, de dyspnée et de bronchospasme ont été rapportés.

Le traitement par un agent sympatholytique, comme la guanéthidine, entraîne de l'hypérémie conjonctivale et de l'irritation, outre le fait que ces agents n'ont qu'une faible propension à réduire la pression intraoculaire.

Enfin, dans le traitement du glaucome par les inhibiteurs de l'anhydrase carbonique, tels que l'acétazolamide ou le méthazolamide, on rapporte de sérieux effets secondaires systémiques tels que la dépression du système nerveux central, la perte de poids et principalement l'hypofonctionnement de la moelle osseuse.

Il apparaît donc clairement que l'utilisation des agents hypotenseurs classiques pour le traitement du glaucome s'accompagne de risques importants. Les médications connues ne sont pas particulièrement bien adaptées pour

un traitement local et les effets secondaires systémiques de ces médicaments en rendent l'emploi délicat parce que ces effets sont loin d'être négligeables et parce qu'ils peuvent dans certains cas avoir des conséquences graves.

Il existe donc un réel besoin de trouver de nouveaux médicaments capables d'abaisser efficacement la tension intraoculaire et qui soient en même temps dépourvus des effets secondaires systémiques évoqués ci-dessus, en particulier lorsqu'ils s'agit de traiter des patients dits "à risques", tels que les cardiaques et les asthmatiques.

La demanderesse a présentement découvert que les nouveaux 2-hydroxy-3-[1-(1H-imidazol-4-yl)alkyl]-benzènecarboximidamides substitués de la présente invention répondent parfaitement à ce besoin, ressenti depuis longtemps. En effet, ces composés sont de puissants agonistes des récepteurs $\alpha_2$-adrénergiques présynaptiques. De plus, ces composés sont dépourvus d'effets secondaires systémiques d'origine centrale ou périphérique. En effet, aux doses où ces composés sont efficaces pour réduire la pression intraoculaire, on n'observe ni hypotension, ni bradycardie, ni mydriase. En outre, ces composés ne provoquent pas d'hypohémie de l'oeil traité et on n'observe pas non plus d'effet secondaire contralatéral de l'oeil non traité lors du traitement d'un oeil par application topique, ce qui démontre bien l'absence de relais, soit par la circulation sanguine, soit par le système neuronal. Les risques associés à l'utilisation des agents thérapeutiques classiques étant pratiquement inexistants avec les composés de l'invention, il en résulte que les 2-hydroxy-3-[1-(1H-imidazol-4-yl)alkyl]-benzènecarboximidamides substitués conformes à la présente invention sont particulièrement bien adaptés pour le traitement d'une hypertension intraoculaire et en particulier du glaucome.

Plus particulièrement, la présente invention se rapporte à de nouveaux 2-hydroxy-3-[1-(1H-imidazol-4-yl)alkyl]-benzènecarboximidamides substitués répondant à la formule générale:

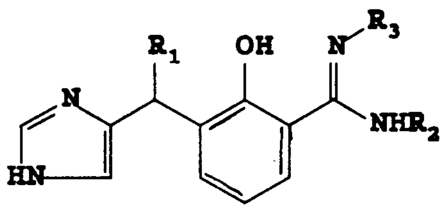

(I)

dans laquelle

- $R_1$ représente un atome d'hydrogène ou un radical alkyle ayant de 1 à 4 atomes de carbone,
- $R_2$ représente un atome d'hydrogène, un groupe hydroxyle, un groupe amino ou un radical alkyle ayant de 1 à 4 atomes de carbone,
- $R_3$ représente un atome d'hydrogène, ou
- $R_2$ et $R_3$ pris ensemble représentent le groupe $-CH_2-CH_2-$,

ainsi que leurs sels d'addition d'acides non toxiques ophtalmologiquement acceptables.

Lorsque la molécule comporte un atome de carbone asymétrique, les composés de formule I peuvent se présenter soit sous la forme d'un mélange racémique, soit sous la forme de l'un ou l'autre énantiomère. Ces diverses formes entrent également dans le cadre de la présente invention.

La présente invention concerne également les sels d'addition d'acides non toxiques ophtalmologiquement acceptables des 2-hydroxy-3-[1-(1H-imidazol-4-yl)alkyl]-benzènecarboximidamides substitués de formule I. On peut employer n'importe quel sel d'addition d'acide pour autant qu'il soit peu toxique et non irritant pour l'oeil. Des exemples d'acides ophtalmologiquement acceptables sont cités à la page 2 du Journal of Pharm. Sciences 66(1), Jan.1977; on peut mentionner entre autres l'acide phosphorique, l'acide maléique, l'acide borique, l'acide carbonique, etc...

Les composés préférés conformes à l'invention sont:

- le 2-hydroxy-3-(1H-imidazol-4-ylméthyl)-benzènecarboximidamide;
- le N,2-dihydroxy-3-(1H-imidazol-4-ylméthyl)-benzènecarboximidamide;
- le 2-hydroxy-3-(1H-imidazol-4-ylméthyl)-N-méthyl-benzènecarboximidamide;
- le 2-hydroxy-3-(1H-imidazol-4-ylméthyl)-N-(1-méthyléthyl)-benzènecarboximidamide;
- l'hydrazide de l'acide 2-hydroxy-3-(1H-imidazol-4-ylméthyl)-benzènecarboximidique;
- le (+)-2-hydroxy-3-[1-(1H-imidazol-4-yl)éthyl]-benzènecarboximidamide; et
- le (-)-2-hydroxy-3-[1-(1H-imidazol-4-yl)éthyl]-benzènecarboximidamide.

Les 2-hydroxy-3-[1-(1H-imidazol-4-yl)alkyl]-benzènecarboximidamides substitués de formule I peuvent être préparés par un procédé général qui comporte les stades suivants:

(1) on fait réagir un 2-hydroxy-3-[1-(1H-imidazol-4-yl)alkyl]-benzonitrile de formule II avec un alcanol ayant de 1 à 4 atomes de carbone en présence d'acide chlorhydrique gazeux selon l'équation:

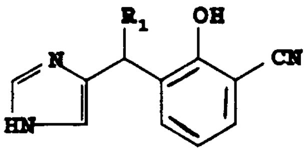

(II)

+ alcanol en $C_1$-$C_4$

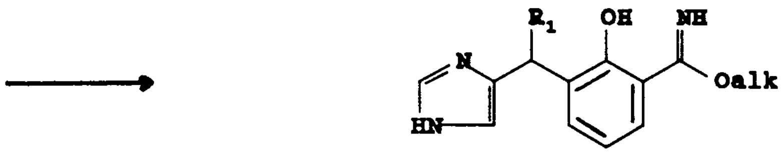

(III)

Cette réaction est généralement effectuée à une température comprise entre -45°C et +15°C.

(2) on fait réagir ensuite le 2-hydroxy-3-[1-(1H-imidazol-4-yl)alkyl]-benzènecarboximidate d'alkyle ainsi obtenu de formule III avec un des énantiomères de l'α-méthylbenzylamine, selon l'équation:

(III) +

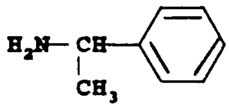

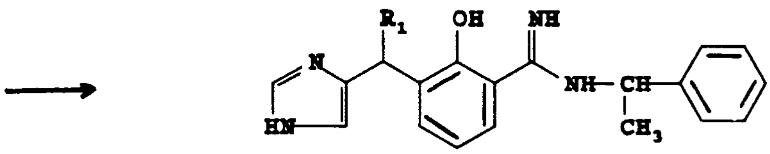

(IV)

(3) on fait réagir finalement le 2-hydroxy-3-[1-(1H-imidazol-4-yl)alkyl]-N-α-méthylbenzyl-benzènecarboximidamide de formule IV obtenu au stade précédent avec un composé azoté de formule $R_2$-$NH_2$ (V) dans laquelle $R_2$ représente un atome d'hydrogène, un groupe hydroxyle, un groupe amino ou un radical alkyle ayant de 1 à 4 atomes de carbone lorsque $R_3$ est de l'hydrogène ou avec l'éthylènediamine (VI) lorsque $R_2$ et $R_3$, pris ensemble, représentent le groupe $-CH_2$-$CH_2$-, selon l'équation:

$$(IV) \quad + \quad R_2\text{-}NH_2 \quad (V) \quad \longrightarrow \quad (I)$$

$$\text{ou}$$

$$NH_2CH_2CH_2NH_2 \quad (VI)$$

dans toutes ces formules, $R_1$ ayant la signification donnée plus haut et alk représentant un radical alkyle ayant de 1 à 4 atomes de carbone.

Il est évident que pour obtenir les composés répondant à la formule générale I sous forme d'un isomère optique, on sépare au préalable les dérivés N-$\alpha$-méthylbenzylés diastéréoisomères de formule IV, dans laquelle $R_1$ représente un radical alkyle ayant de 1 à 4 atomes de carbone, avant de procéder à la réaction d'aminolyse au stade (3).

Ce procédé général convient donc éminemment bien pour préparer tous les composés répondant à la formule générale I, que ce soit sous la forme racémique ou sous la forme d'un isomère optique lorsque $R_1$ représente un radical alkyle ayant de 1 à 4 atomes de carbone ou encore sous une forme optiquement inactive lorsque la molécule ne comporte pas de carbone asymétrique ($R_1$ = hydrogène).

Selon une forme de réalisation particulière de préparation des 2-hydroxy-3-[1-(1H-imidazol-4-yl)alkyl]-benzènecarboximidamides répondant à la formule générale I, on synthétise d'abord un 2-hydroxy-3-[1-(1H-imidazol-4-yl)alkyl]-benzènecarboximidate d'alkyle de formule III en exécutant uniquement le stade (1) du procédé décrit ci-dessus et on fait réagir ensuite ce benzènecarboximidate d'alkyle de formule III avec un composé azoté de formule $R_2$-$NH_2$ (V) dans laquelle $R_2$ représente un atome d'hydrogène, un groupe hydroxyle, un groupe amino ou un radical alkyle ayant de 1 à 4 atomes de carbone lorsque $R_3$ est de l'hydrogène ou avec l'éthylènediamine (VI) lorsque $R_2$ et $R_3$, pris ensemble, représentent le groupe $-CH_2$-$CH_2$-.

Selon une variante, réservée à la préparation des 2-hydroxy-3-[1-(1H-imidazol-4-yl)alkyl]-benzènecarboximidamides répondant à la formule générale I dans laquelle $R_2$ représente un groupe hydroxyle et $R_3$ représente un atome d'hydrogène, on fait réagir l'hydroxylamine avec un 2-hydroxy-3-[1-(1H-imidazol-4-yl)alkyl]-benzonitrile de formule II dans laquelle $R_1$ à la signification donnée plus haut.

Dans le cas particulier de la préparation des 2-hydroxy-3-[1-(1H-imidazol-4-yl)alkyl]-benzènecarboximidamides répondant à la formule générale I dans laquelle $R_1$ représente un radical alkyle ayant de 1 à 4 atomes de carbone, et $R_2$ et $R_3$ représentent tout deux un atome d'hydrogène, sous forme de leurs isomères optiques, on synthétise d'abord un 2-hydroxy-3-[1-(1H-imidazol-4-yl)alkyl]-N-$\alpha$-méthylbenzyl-benzènecarboximidamide de formule IV en exécutant uniquement les stades (1) et (2) du procédé décrit ci-dessus, on sépare les dérivés N-$\alpha$-méthylbenzylés diastéréoisomères de formule IV obtenus et on élimine ensuite le groupe $\alpha$-méthylbenzyle de chaque diastéréoisomère ainsi séparé, par hydrolyse au moyen d'acide chlorhydrique concentré à une température comprise entre 80°C et 110°C.

Les sels d'additions d'acides non toxiques ophtalmologiquement acceptables peuvent être préparés à partir des 2-hydroxy-3-[1-(1H-imidazol-4-yl)alkyl]-benzènecarboximidamides de formule I par des méthodes connues en soi.

Les 2-hydroxy-3-[1-(1H-imidazol-4-yl)alkyl]-benzonitriles de formule II utilisés comme produits de départ, peuvent être préparés par le procédé décrit dans le brevet européen n° 341.231.

Comme il a été indiqué plus haut, les 2-hydroxy-3-[1-(1H-imidazol-4-yl)alkyl]-benzènecarboximidamides substitués de formule I, ainsi que leurs sels d'addition d'acides non toxiques ophtalmologiquement acceptables possèdent des propriétés agonistes des récepteurs $\alpha_2$-adrénergiques pré-synaptiques, ils sont capables d'abaisser la tension intraoculaire, et sont dépourvus d'effets secondaires importants.

Les essais pharmacologiques décrits ci-après mettent en évidence ces diverses propriétés avantageuses.

Les composés suivants ont été soumis à des essais pharmacologiques:

- 2-hydroxy-3-(1H-imidazol-4-ylméthyl)-benzènecarboximidamide (composé A, préparé à l'exemple 2.1);
- 2-hydroxy-3-(1H-imidazol-4-ylméthyl)-N-méthyl-benzènecarboximidamide (composé B, préparé à l'exemple 2.2);
- N,2-dihydroxy-3-(1H-imidazol-4-ylméthyl)-benzènecarboximidamide (composé C, préparé à l'exemple 3);
- (+)-2-hydroxy-3-[1-(1H-imidazol-4-yl)éthyl]-benzènecarboximidamide (composé D, préparé à l'exemple 4);
- (-)-2-hydroxy-3-[1-(1H-imidazol-4-yl)éthyl]-benzènecarboximidamide (composé E, préparé à l'exemple 4);
- 2-hydroxy-3-(1H-imidazol-4-ylméthyl)-N-(1-méthyléthyl)-benzènecarboximidamide (composé F, préparé à l'exemple 5.2);
- hydrazide de l'acide 2-hydroxy-3-(1H-imidazol-4-ylméthyl)-benzènecarboximidique (composé G, préparé à l'exemple 2.6).

Les résultats ont été comparés à ceux obtenus avec la clonidine, un agoniste des récepteurs $\alpha_2$-adrénergiques qui, comme certains de ses dérivés, est utilisé dans le traitement du glaucome.

1. Propriété agoniste $\alpha_2$-adrénergique pré-synaptique.

Stimulation de l'iléon de cobaye.

La propriété agoniste $\alpha_2$-adrénergique pré-synaptique des composés de l'invention est mise en évidence par la mesure de l'inhibition de la contraction de l'iléon isolé de cobaye provoquée par une stimulation électrique.

Des fragments de muscles longitudinaux attachés à un indicateur de force isométrique, sont plongés dans une solution de Tyrode et sont tendus avec une force de 1 g (G.M. DREW, Brit.J.Pharmacol. 64, (1978), 293-300; M. ANDREJAK et coll. Naunyn-Schmiedeberg's Arch.Pharmacol. 314, (1980), 83-87).

La stimulation électrique des nerfs parasympathiques afférents aux fragments d'iléon provoque une contraction du muscle. Cette contraction est réduite en présence d'un $\alpha_2$-agoniste pré-synaptique et la réduction dépend de la concentration de l'agoniste utilisée. Cet effet est antagonisé par la présence simultanée d'un composé $\alpha_2$-antagoniste tel que l'alpha-yohimbine.

Les composés à étudier ont été testés à des concentrations croissantes comprises entre $10^{-10}$ et $10^{-3}$ mole/l. On détermine la concentration ($IC_{50}$ en mole/l) qui réduit de 50% l'intensité de la contraction du muscle.

Dans le tableau I, on indique les concentrations $IC_{50}$ (en mole/l) obtenues pour les composés de l'invention, sauf pour le composé G pour lequel on indique la concentration $IC_{30}$. Ces résultats montrent que ces composés sont bien actifs à très faible concentration.

TABLEAU I

| Inhibition de la contraction de l'iléon de cobaye. | |
|---|---|
| Composé | $IC_{50}$ (en mole/l) |
| A | $6,2 \times 10^{-8}$ |
| B | $2,5 \times 10^{-6}$ |
| C | $1,4 \times 10^{-7}$ |
| D | $3,9 \times 10^{-9}$ |
| E | $2,0 \times 10^{-7}$ |
| F | $6,4 \times 10^{-6}$ |
| G | $2,5 \times 10^{-6}$ ($IC_{30}$) |
| clonidine | $1,7 \times 10^{-8}$ |

2. Activité hypotensive intraoculaire.

L'effet d'abaissement de la pression intraoculaire des composés est mis en évidence chez le lapin (New Zealand White) normotendu éveillé, par la mesure de la variation de la pression intraoculaire après application unilatérale des composés dans l'oeil gauche de l'animal. Trois lapins (poids: 2-2,5 kg) de chaque sexe reçoivent toutes les doses testées selon un protocole expérimental en "permutation-croisée" dans lequel chaque animal sert aussi au groupe contrôle. Les composés à étudier, de même que la clonidine sont administrés en solution, préalablement laissée à température ambiante pendant 30 minutes, dans 30 microlitres d'eau distillée stérile (collyre) à des concentrations s'échelonnant entre 0 (référence) et 0,5 % (en poids par volume). Les mesures de pression intraoculaire sont réalisées à l'aide d'un pneumatomètre BIORAD (DIGILAB MODULAR ONE) après anesthésie locale au moyen de 2 gouttes de chlorhydrate d'oxybuprocaïne à 0,4 % (en poids par volume).

Le tableau II ci-dessous montre les variations de pression observées lors de l'instillation des composés de l'invention ainsi que de la clonidine. Dans ce tableau, on indique,

dans la 1$^{ère}$ colonne, le composé testé,
dans la 2$^{e}$ colonne, la concentration du composé, en pour-cent (en poids par volume),
dans les 3$^{e}$, 4$^{e}$, 5$^{e}$, 6$^{e}$ et 7$^{e}$ colonnes, la variation de la pression intraoculaire ($\Delta P$), exprimée en pour-cent, par rapport à un animal traité avec un collyre dépourvu de composé à tester, mesurée après 30 minutes, 1, 2, 3 ou 6 heures respectivement.

TABLEAU II

| Réduction de la pression intraoculaire. | | | | | | |
|---|---|---|---|---|---|---|
| Composé | Conc. (%) | ΔP [30 min] (%) | ΔP [1 h] (%) | ΔP [2h] (%) | ΔP [3h] (%) | ΔP [6h] (%) |
| A | 0,05 | - 5,9 | | - 18,6 | | - 1,1 |
| | 0,1 | - 9,2 | | - 21,2 | | - 16,1 |
| B | 0,5 | -5,5 | -10,8 | -13,3 | -12,8 | -7,6 |
| D | 0,01 | - 14,1 | | - 17,7 | | - 15,1 |
| E | 0,5 | - 11,6 | - 12,6 | | | - 6,6 |
| clonidine | 0,01 | + 8 | | - 8,8 | | - 9,2 |
| | 0,1 | + 23,4 | | + 36,2 | + 11,4 | |
| | 0,5 | + 31,4 | | + 39,4 | + 8,6 | |

Il ressort de ce tableau que les composés de l'invention possèdent une bonne activité hypotensive intraoculaire, contrairement à la clonidine qui provoque initialement une hypertension de l'oeil traité, dont l'effet augmente en même temps que la concentration du produit utilisé. Cet effet biphasique n'est pas observé avec les composés conformes à l'invention.

En outre, pour les composés de l'invention, on n'observe pas de baisse concomitante de la pression intraoculaire dans l'oeil contralatéral des animaux traités. Par contre, avec la clonidine, on mesure les variations de pression intraoculaire suivantes dans l'oeil contralatéral:

| | |
|---|---|
| - à la concentration de 0,01 %, ΔP = | - 9,5 % après 2 heures et |
| | - 9,2 % après 6 heures; |
| - a la concentration de 0,1 %, ΔP = | - 10,2 % après 2 heures et |
| | + 2,6 % après 6 heures. |
| - a la concentration de 0,5 %, ΔP = | - 26,3 % après 2 heures et |
| | + 15 % après 6 heures. |

Ceci met en évidence l'absence de relais, soit par la circulation sanguine, soit par le système neuronal des composés de l'invention. En outre, ces résultats montrent la difficulté d'obtenir une baisse de la tension intraoculaire avec la clonidine en raison de son effet biphasique.

3. Effets sur le diamètre pupillaire de l'oeil traité et de l'oeil contralatéral.

L'effet des composés de l'invention sur le diamètre de la pupille est mis en évidence chez le lapin (New Zealand White) des deux sexes (poids 2-2,5 kg) normotendu éveillé, par la mesure de la variation du diamètre de la pupille des deux yeux après application topique des composés sur l'oeil gauche de l'animal. Les composés à étudier de même que la clonidine ont été administrés en solution, préalablement laissée à température ambiante pendant 30 minutes, dans 30 microlitres d'eau distillée stérile (collyre) à des concentrations s'échelonnant entre 0 (référence) et 0,5 % (en poids par volume). Le diamètre de la pupille est mesuré visuellement au point du diamètre vertical maximal à l'aide d'un étalon millimétrique en métal.

Les composés de l'invention ne provoquent pas ou peu de modification significative du diamètre pupillaire aux doses où ils diminuent la pression oculaire (tableau II), quel que soit l'oeil observé (traité ou contralatéral). En effet, les écarts maximaux entre le diamètre pupillaire mesuré avant instillation du composé à tester et le diamètre pupillaire mesuré à des temps d'observation compris entre 30 minutes et 6 heures après instillation du composé à tester s'étalent entre - 4,2 % à + 4,7 %.

Le tableau III montre les résultats observés dans les mêmes conditions avec la clonidine. Dans ce tableau, on donne, dans la 1$^{ère}$ colonne, la concentration en clonidine, en pour-cent (poids/volume);
dans la 2$^{e}$ colonne, l'écart maximal observé entre le diamètre pupillaire de l'oeil traité (ipsilatéral) mesuré avant instillation de la clonidine et le diamètre pupillaire du même oeil mesuré à des temps d'observation compris entre 30 minutes et 6 heures après instillation;

dans la 3e colonne, l'écart maximal observé dans les mêmes conditions sur le diamètre de la pupille de l'oeil contralatéral.

TABLEAU III

| Effet de la clonidine sur le diamètre pupillaire. | | |
|---|---|---|
| Concentration en clonidine (%) | Ecart maximal (oeil ipsilatéral) (%) | Ecart maximal (oeil contralatéral) (%) |
| 0,01 | - 5,6 | - 2,8 |
| 0,1 | + 9,7 | - 2,7 |
| 0,5 | + 23,9 | - 6,9 |

Ce tableau montre que, contrairement aux composés de l'invention, une mydriase importante de l'oeil ipsilatéral s'observe avec la clonidine, aux concentrations (0,1 et 0,5 %) où l'on n'a pas de baisse de la pression intraoculaire.

4. Effets sur la muqueuse de l'oeil traité (hypohémie).

L'effet des composés de l'invention sur la muqueuse de l'oeil traité est mis en évidence chez le lapin (New Zealand White) des deux sexes (poids 2-2,5 kg) normotendu éveillé, par l'examen visuel des conjonctives afin de détecter des signes éventuels de modification ou de réaction au traitement après application topique des composés sur l'oeil de l'animal. L'hypohémie se manifeste par une irrigation sanguine insuffisante de la conjonctive pouvant entraîner des ischémies locales irréversibles. Les composés à étudier ont été administrés comme dans les tests précédents en solution dans 30 microlitres d'eau distillée stérile (collyre) à des concentrations s'échelonnant entre 0 (référence) et 0,5 % (en poids par volume).

Aux doses efficaces pour abaisser la pression intraoculaire, par exemple 0,1 % pour le composé A ou 0,5% pour le composé E, les composés de l'invention ne provoquent pas d'hypohémie. Par contre, pour la clonidine à la concentration de 0,01 %, on observe un blanchiment des conjonctives après 30 minutes; cet effet est encore présent après 1 heure pour la clonidine à la concentration de 0,1 %.

5. Effets sur la fréquence cardiaque.

L'effet sur la fréquence cardiaque des composés est mis en évidence chez le lapin (New Zealand White) des deux sexes (poids 2-2,5 kg) normotendu éveillé, par la mesure des battements cardiaques au niveau de l'artère caudale après application topique des composés sur l'oeil gauche de l'animal. Les composés à étudier ont été administrés en solution dans 30 microlitres d'eau distillée stérile (collyre) à des concentrations s'échelonnant entre 0 (référence) et 0,5 % (en poids par volume).

On n'observe pas d'effets significatifs sur la fréquence cardiaque chez les animaux traités avec les composés de l'invention. Par contre, chez les animaux traités avec la clonidine à la concentration de 0,01 %, on observe un ralentissement sensible des battements cardiaques de 10,7 % après 3 heures et, chez les animaux traités avec la clonidine à la concentration de 0,1 %, on observe un abaissement important de la fréquence cardiaque de 17,9 % après 1 heure.

6. Toxicité.

La toxicité des composés de l'invention a été déterminée chez la souris mâle NMRI au moyen du test d'Irwin (S.IRWIN, Psychopharmacologia, 13, (1968), 222-257). Des doses progressives du produit étudié sont administrées par voie intrapéritonéale à des groupes de trois souris jusqu'à ce que la dose mortelle soit atteinte (dose provoquant la mort de deux animaux sur trois dans les 24 heures).

Dans le tableau IV ci-dessous, on donne la dose mortelle observée pour les composés de l'invention. Il ressort de ce tableau que ces composés sont très peu toxiques.

TABLEAU IV

| Toxicité. | |
|---|---|
| Composé | Dose mortelle (en mg/kg) |
| A | >216 |
| B | 230 |
| C | 232 |
| D | 25 |
| E | 248 |
| F | 155 |
| G | 416 |

Les composés de la présente invention sont administrés de préférence sous la forme d'une composition pharmaceutique ophtalmique adaptée à l'administration topique dans l'oeil, par exemple sous forme de solutions ou pommades ou encore sous forme d'un implant solide applicable sur l'oeil. Le pourcentage de produit actif dans les compositions pharmaceutiques peut varier de 0,01 à 1 %, et de préférence, de 0,05 à 0,5 % en poids. Quant à la posologie journalière, les composés de l'invention sont généralement administrés dans l'oeil en une dose comprise entre 1 µg et 1 mg et de préférence entre 50 µg et 0,5 mg, de produit actif, seul ou en mélange.

Dans les compositions pharmaceutiques ophtalmiques les composés de la présente invention peuvent être commodément associés avec un ou plusieurs excipients ophtalmologiquement acceptables non toxiques, solides ou liquides. Des excipients ophtalmologiquement acceptables sont par exemple l'eau, les mélanges d'eau et de solvants miscibles à l'eau, tels que les alcools aliphatiques ou araliphatiques inférieurs, les huiles végétales, les polyalkylèneglycols, l'éthylcellulose, l'oléate d'éthyle, la carboxyméthylcellulose, la polyvinylpyrrolidine, le myristate d'isopropyle ou encore d'autres excipients habituels. Les compositions ophtalmiques peuvent en outre contenir des substances auxiliaires non toxiques, telles que des émulsifiants, des agents de conservation, des agents mouillants, etc., comme par exemple les polyéthylèneglycols 200, 300, 400, 600, 1000, 1500, 4000, 6000 et 10.000, des agents bactéricides, comme les ammoniums quaternaires ou les sels de phénylmercure, connus pour avoir des propriétés de stérilisation à froid et pour ne pas présenter d'effets nocifs, le méthylparaben, le propylparaben, l'alcool benzylique, le 2-phényléthanol, des tampons à base de chlorure de sodium, de borate de sodium, d'acétate de sodium, les tampons gluconates, etc.

En outre, des excipients ophtalmologiquement acceptables liquides appropriés peuvent être utilisés. Des exemples de ces excipients liquides sont les solutions tampons à base de phosphate, les solutions isotoniques d'acide borique, de chlorure de sodium, de borate de sodium, etc..

Les compositions ophtalmiques peuvent aussi se présenter sous forme d'un implant solide que l'on applique sur l'oeil. Dans ce cas, on peut employer par exemple un polymère solide soluble dans l'eau comme support pour le médicament. Le polymère utilisé pour former l'implant peut être n'importe quel polymère non toxique soluble dans l'eau, par exemple des dérivés de la cellulose, comme la méthylcellulose, la carboxyméthylcellulose de sodium, des hydroxyalkylcelluloses avec des alkyls inférieurs, comme l'hydroxyéthylcellulose, l'hydroxypropylcellulose, l'hydroxypropylméthylcellulose, des acrylates, comme l'acide polyacrylique ou les polyacrylamides, des produits naturels, comme la gélatine, les alginates, les pectines, la gomme Tragacanth, des dérivés de l'amidon, comme l'acétate d'amidon, l'hydroxyéthylamidon, l'hydroxypropylamidon, de même que d'autres dérivés synthétiques comme l'alcool polyvinylique, la polyvinylpyrrolidone, le polyvinylméthyléther, l'oxyde de polyéthylène et les mélanges desdits polymères.

Dans le cas où on utilise un implant solide à appliquer sur l'oeil, on prépare de préférence celui-ci à partir de dérivés de la cellulose, tels que la méthylcellulose, l'hydroxyéthylcellulose, l'hydroxypropylcellulose ou l'hydroxypropylméthylcellulose ou à partir d'autres matériaux synthétiques, tels que l'alcool polyvinylique, la polyvinylpyrrolidone, l'oxyde de polyéthylène ou le polyvinylméthyléther.

A titre d'exemple non limitatif d'une composition contenant un composé de l'invention, on donne ci-après un exemple d'une solution stérile pour application topique sous forme de collyre:

| ingrédients | quantités (% en p/v) |
|---|---|
| composé actif | 0,01 à environ 1 % |
| alcool polyvinylique | 0 à 40 % |
| chlorure de benzalkonium | 0 à 0,15 % |
| chlorure de sodium | 0 à 10 % |
| tampon | 0,01 à 10 % |
| eau distillée stérile | q.s. ad 100 % |

Les exemples qui suivent illustrent l'invention sans toutefois la limiter. Dans ces exemples, les points de fusion ont été déterminés par calorimétrie à balayage différentiel (D.S.C.) avec un gradient de température de 20°C/min. Les spectres de résonance magnétique nucléaire (RMN) ont été relevés avec un appareil Bruker à 250 MHz dans le diméthylsulfoxyde en utilisant le tétraméthylsilane comme étalon interne. Les déplacements chimiques sont indiqués en δ (ppm). Les lettres s, d, dd, t, q, b et m indiquent respectivement un singulet, un doublet, un double doublet, un triplet, un quadruplet, un pic élargi et un multiplet.

Exemple 1. Préparation des 2-hydroxy-3-[1-(1H-imidazol-4-yl)alkyl]-benzonitriles de départ de formule II.

1. 2-hydroxy-3-(1H-imidazol-4-ylméthyl)-benzonitrile.

On prépare ce composé selon la méthode décrite à l'exemple 3.1 du brevet européen 341.231.

2. 2-hydroxy-3-[1-(1H-imidazol-4-yl)éthyl]-benzonitrile.

On prépare ce composé selon la méthode décrite à l'exemple 3.2 du brevet européen 341.231.

Exemple 2. Préparation de 2-hydroxy-3-[1-(1H-imidazol-4-yl)alkyl]-benzènecarboximidamides substitués de formule I.

1. 2-hydroxy-3-(1H-imidazol-4-ylméthyl)-benzènecarboximidamide.

a) On refroidit à -40°C une suspension de 10 g (0,050 mole) de 2-hydroxy-3-(1H-imidazol-4-ylméthyl)-benzonitrile (préparé à l'exemple 1.1 ci-dessus) dans 200 ml de méthanol et on la sature par de l'acide chlorhydrique gazeux. On laisse la température revenir entre 0 et 5°C et on maintient le mélange réactionnel à cette température pendant 20 heures. On évapore la solution aux 9/10 de son volume, on ajoute au résidu 300 ml d'eau glacée et on neutralise au moyen d'une solution aqueuse de bicarbonate de sodium. On extrait la solution à 4 reprises avec 150 ml d'acétate d'éthyle et on réunit les phases organiques. On lave la phase organique avec 100 ml d'une solution aqueuse saturée en chlorure de sodium, puis on sèche sur sulfate de magnésium, on filtre et on évapore sous pression réduite.

b) On reprend le résidu obtenu par 150 ml d'éthanol absolu. On refroidit la solution en dessous de 10°C et on sature par de l'ammoniac. On laisse la solution revenir à la température ambiante et on maintient le mélange réactionnel à cette température pendant 15 heures. On évapore le solvant sous pression réduite. On purifie le résidu solide par chromatographie liquide préparative (silice: 600 g; éluant: mélange 77,5:20:2,5 (v/v/v) dichlorométhane - méthanol - ammoniaque). On isole 8 g d'un produit solide blanc qu'on recristallise dans du méthanol. On obtient 4,55 g de 2-hydroxy-3-(1H-imidazol-4-ylméthyl)-benzènecarboximidamide. Rendement: 42%.

P.F.: 248,71°C.
RMN: δ: 3,68 (2H,s), 6,13 (1H,dd), 6,61 (1H,s), 6,95 (1H,dd), 7,41 (1H,d), 7,43 (1H,dd)

| Analyse pour $C_{11}H_{12}N_4O$ en % : | | | |
|---|---|---|---|
| calculé: | C 61,10 | H 5,59 | N 25,91 |
| trouvé: | 61,18 | 5,62 | 26,07 |

2. 2-hydroxy-3-(1H-imidazol-4-ylméthyl)-N-méthyl-benzènecarboximidamide.

a) On sature par de l'acide chlorhydrique gazeux une suspension de 19,9 g (0,1 mole) de 2-hydroxy-3-(1H-imidazol-4-ylméthyl)benzonitrile (préparé à l'exemple 1.1 ci-dessus) dans 600 ml de méthanol refroidi à 10°C. On y ajoute 60 ml d'eau, on refroidit la suspension à -25°C et on la sature une nouvelle fois par de l'acide chlorhydrique gazeux. On maintient le mélange réactionnel à cette température pendant 15 heures. On évapore la solution et le résidu est repris par 500 ml d'eau glacée et on neutralise au moyen d'une solution aqueuse de bicarbonate de sodium. On extrait la solution à 4 reprises avec 250 ml d'acétate d'éthyle et on réunit les phases organiques. On lave la phase organique avec 200 ml d'une solution aqueuse saturée en chlorure de sodium, puis on sèche sur sulfate de magnésium, on filtre et on évapore. On obtient 19,1 g de 2-hydroxy-3-(1H-imidazol-4-ylméthyl)-benzènecarboximidate de méthyle qui est utilisé tel quel dans l'étape suivante.
b) On dissout 9,55 g (0,0413 mole) de 2-hydroxy-3-(1H-imidazol-4-ylméthyl)-benzènecarboximidate de méthyle dans 100 ml d'éthanol absolu, on refroidit la solution à 10°C et on y ajoute 1,28 g (0,0413 mole) de méthylamine. On laisse la solution revenir à la température ambiante et on laisse réagir pendant 15 heures. On évapore le solvant sous pression réduite. Le résidu est purifié par chromatographie liquide préparative (silice: 600 g; éluant: mélange 78:20:2 (v/v/v) acétate d'éthyle - méthanol - ammoniaque). On isole 9,49 g de produit que l'on agite dans 100 ml d'acétonitrile à 50°C pendant 30 minutes. On filtre le produit solide et on le recristallise dans du méthanol. On obtient 4,5 g de 2-hydroxy-3-(1H-imidazol-4-ylméthyl)-N-méthyl-benzènecarboximidamide. Rendement: 47%.

P.F.: 235,84°C.
RMN: δ: 2,94 (3H,s), 3,70 (2H,s), 6,20 (1H,dd), 6,60 (1H,s), 6,95 (1H,dd), 7,41 (1H,d), 7,47 (1H,dd), 8,47 (2H,m), 14,1 (1H,m)

| Analyse pour $C_{12}H_{14}N_4O$ en % : | | | |
|---|---|---|---|
| calculé: | C 62,59 | H 6,15 | N 24,73 |
| trouvé: | 62,53 | 6,15 | 24,30 |

3. 2-(4,5-dihydro-1H-imidazol-2-yl)-6-(1H-imidazol-4-ylméthyl)-phénol.

a) Le 2-hydroxy-3-(1H-imidazol-4-ylméthyl)-benzènecarboximidate de méthyle est préparé comme en 2.a) ci-dessus et est utilisé tel quel dans l'étape suivante.
b) On dissout 9,55 g (0,0413 mole) du produit préparé en 3.a) ci-dessus dans 100 ml d'éthanol absolu et on y ajoute 6,65 ml (0,0826 mole) d'éthylènediamine. On agite la solution pendant une heure à la température ambiante puis on laisse réagir pendant 15 heures. On filtre le précipité qui s'est formé (1er jet). Le filtrat est évaporé sous pression réduite et le résidu obtenu est purifié par chromatographie liquide préparative (silice: 400 g; éluant: mélange 78:20:2 (v/v/v) acétate d'éthyle - méthanol - ammoniaque). On isole 1,7 g d'un composé solide blanc (2ème jet). On réunit les deux jets et on recristallise deux fois dans du méthanol. On obtient 3,3 g de 2-(4,5-dihydro-lH-imidazol-2-yl)-6-(1H-imidazol-4-ylméthyl)-phénol. Rendement: 33%.

P.F.: 260,92°C.
RMN: δ: 3,71 (3H,s), 3,77 (2H,s), 6,53 (1H,t), 6,64 (1H,s), 7,07 (1H,d), 7,40 (1H,dd), 7,45 (1H,s), 11,1 (2H,m),

11,7 (1H,m)

| Analyse pour $C_{13}H_{14}N_4O$ en % : | | | |
|---|---|---|---|
| calculé: | C 64,46 | H 5,82 | N 23,13 |
| trouvé: | 64,46 | 5,86 | 23,14 |

4. 2-hydroxy-3-[1-(1H-imidazol-4-yl)éthyl]-benzènecarboximidamide.

a) On refroidit à -40°C une suspension de 10 g (0,047 mole) de 2-hydroxy-3-[1-(1H-imidazol-4-yl)éthyl]-benzonitrile (préparé à l'exemple 1.2 ci-dessus) dans 100 ml de méthanol et on la sature par de l'acide chlorhydrique gazeux. On laisse la température revenir jusqu'à 5°C et on maintient le mélange réactionnel à cette température pendant 24 heures. On évapore la solution à froid aux 3/4 de son volume, on ajoute au résidu 200 ml d'eau et 150 ml d'acétate d'éthyle et on neutralise au moyen d'une solution aqueuse de bicarbonate de sodium. On extrait la solution à 3 reprises par 100 ml d'acétate d'éthyle et on réunit les phases organiques. On lave la phase organique avec 100 ml d'une solution aqueuse saturée en chlorure de sodium puis on sèche sur sulfate de magnésium, on filtre et on évapore le solvant sous pression réduite à la température ambiante.

b) On reprend le résidu obtenu par 50 ml d'éthanol absolu, on refroidit la solution en dessous de 10°C et on sature par de l'ammoniac gazeux. On laisse la solution revenir à la température ambiante et on maintient le mélange réactionnel à cette température pendant 6 heures. Ensuite, on évapore le solvant sous pression réduite. Le résidu obtenu est purifié par chromatographie liquide préparative (silice: 500 g; éluant: mélange 78:20:2 (v/v/v) dichlorométhane - méthanol - ammoniaque). Le produit obtenu après évaporation des solvants est recristallisé deux fois dans un mélange 90/10 (v/v) eau - méthanol. On obtient 8,75 g de 2-hydroxy-3-[1-(1H-imidazol-4-yl)éthyl]-benzènecarboximidamide. Rendement: 81%.

P.F.: 190,6°C.
RMN: δ: 1,40 (3H,s), 4,48 (1H,q), 6,14 (1H,dd), 6,63 (1H,s), 6,88 (1H,dd), 7,40 (1H,s), 7,41 (1H,dd)

| Analyse pour $C_{12}H_{14}N_4O$ en % : | | | |
|---|---|---|---|
| calculé: | C 62,59 | H 6,13 | N 24,33 |
| trouvé: | 62,58 | 6,12 | 24,37 |

5. N,2-dihydroxy-3-(1H-imidazol-4-ylméthyl)-benzènecarboximidamide.

a) On refroidit à -40°C une suspension de 14,1 g (0,071 mole) de 2-hydroxy-3-(1H-imidazol-4-ylméthyl)-benzonitrile (préparé à l'exemple 1.1 ci-dessus) dans 420 ml de méthanol et on la sature par de l'acide chlorhydrique gazeux. On laisse la température revenir jusqu'à 10°C et on sature une nouvelle fois la suspension par de l'acide chlorhydrique gazeux. On maintient le mélange réactionnel à cette température pendant 20 heures. On concentre la solution jusqu'à 200 ml, on ajoute à la solution concentrée 300 ml d'eau glacée et 300 ml d'acétate d'éthyle et on neutralise au moyen d'une solution aqueuse de bicarbonate de sodium. On filtre et on sépare la phase aqueuse de la phase organique, puis on extrait la phase aqueuse à deux reprises par 300 ml d'acétate d'éthyle et on réunit les trois phases organiques. On lave la phase organique avec une solution aqueuse saturée en chlorure de sodium, puis on sèche sur sulfate de magnésium, on filtre et on évapore le solvant sous pression réduite. On obtient 15,4 g de 2-hydroxy-3-(1H-imidazol-4-ylméthyl)-benzènecarboximidate de méthyle sous forme d'un produit solide beige que l'on reprend dans 210 ml d'éthanol absolu.

b) On ajoute 1,53 g (0,022 mole) de chlorhydrate d'hydroxylamine et 3,06 ml (0,022 mole) de triéthylamine à 70 ml de la solution de 2-hydroxy-3-(1H-imidazol-4-ylméthyl)-benzènecarboximidate de méthyle dans l'éthanol absolu

préparée en 5.a) ci-dessus. On laisse le mélange réactionnel réagir pendant 1 heure à 20°C. On évapore le solvant sous pression réduite et on reprend, sous ultrasons, le résidu huileux dans 100 ml d'eau. On isole 4,5 g d'un produit solide blanc que l'on recristallise deux fois dans du méthanol. On obtient 3,4 g de N,2-dihydroxy-3-(1H-imidazol-4-ylméthyl)-benzènecarboximidamide. Rendement: 62 %.

P.F.: 227,81°C.
RMN: δ: 3,83 (2H,s), 6,24 (2H,s), 6,67 (1H,s), 6,75 (1H,t), 7,05 (1H,d), 7,49 (1H,s), 7,51 (1H,d), 10,07 (1H,m), 11,7 (1H,m), 12,5 (1H,m)

| Analyse pour $C_{11}H_{12}N_4O_2$ en %: | | | |
|---|---|---|---|
| calculé: | C 56,88 | H 5,21 | N 24,13 |
| trouvé: | 56,83 | 5,25 | 24,09 |

6. hydrazide de l'acide 2-hydroxy-3-(1H-imidazol-4-ylméthyl)-benzènecarboximidique.

a) On refroidit à -45°C une suspension de 14,1 g (0,071 mole) de 2-hydroxy-3-(1H-imidazol-4-ylméthyl)-benzonitrile (préparé à l'exemple 1.1 ci-dessus) dans 400 ml de méthanol et on la sature par de l'acide chlorhydrique gazeux. On laisse la température revenir jusqu'à 10°C et on laisse encore barboter de l'acide chlorhydrique gazeux à travers la suspension pendant 4 heures à cette température. On refroidit à 5°C et on maintient la suspension à cette température pendant 20 heures. On filtre le précipité qui s'est formé et on obtient 11,4 g de 2-hydroxy-3-(1H-imidazol-4-ylméthyl)-benzènecarboximidate de méthyle sous forme d'un solide blanc que l'on utilise tel quel dans l'étape suivante.
b) On ajoute en une fois une solution de 2 g (0,0066 mole) de 2-hydroxy-3-(1H-imidazol-4-ylméthyl)-benzènecarboximidate de méthyle préparé en 6.a) ci-dessus dans 20 ml de méthanol à une solution de 1,52 ml (0,0033 mole) d'hydrate d'hydrazine dans 20 ml de méthanol. On laisse réagir pendant 15 minutes, puis on ajoute 40 ml de diéthyléther. On filtre le précipité qui s'est formé et on concentre le filtrat sous pression réduite. Le résidu d'évaporation est purifié par chromatographie liquide préparative (silice: 400 g; éluant: mélange 89:10:1 (v/v/v) dichlorométhane - méthanol - ammoniaque). Le produit solide jaune pâle obtenu après évaporation des solvants est recristallisé dans du méthanol. On obtient 1,2 g d'hydrazide de l'acide 2-hydroxy-3-(1H-imidazol-4-ylméthyl)-benzènecarboximidique. Rendement: 80 %.

P.F.: 187,8°C.
RMN: δ: 3,77 (2H,s), 5,09 (2H,b), 6,34 (2H,b), 6,56 à 6,64 (2H,m), 6,97 (1H,d), 7,44 (2H,m), 11,7 (1H,b), 14,7 (1H,b)

| Analyse pour $C_{11}H_{13}N_5O.½H_2O$ en %: | | | |
|---|---|---|---|
| calculé: | C 54,98 | H 5,87 | N 29,15 |
| trouvé: | 54,96 | 5,89 | 28,39 |

Exemple 3.

Préparation de la N,2-dihydroxy-3-(1H-imidazol-4-ylméthyl)-benzènecarboximidamide.

On dissout 3 g (0,015 mole) de 2-hydroxy-3-(1H-imidazol-4-ylméthyl)-benzonitrile (préparé à l'exemple 1.1) dans 100 ml de méthanol et on y ajoute 1,15 g (0,0165 mole) de chlorhydrate d'hydroxylamine et 1,84 g d'acétate de sodium. Le mélange est chauffé à la température de reflux pendant 20 heures. On évapore le méthanol sous pression réduite et on reprend le résidu par 100 ml d'eau. La solution aqueuse est neutralisée à pH 7 par addition d'une solution aqueuse de bicarbonate de sodium. On filtre le précipité blanc qui s'est formé, on le sèche sous vide et on le recristallise dans du méthanol. On obtient 1,6 g de N,2-dihydroxy-3-(1H-imidazol-4-ylméthyl)-benzènecarboximidamide, composé qui est identique à celui préparé à l'exemple 2.5. Rendement: 46%.

Exemple 4.

Préparation de 2-hydroxy-3-[1-(1H-imidazol-4-yl)éthyl]-benzènecarboximidamides optiquement actifs.

(-)-2-hydroxy-3-[1-(1H-imidazol-4-yl)éthyl]benzènecarboximidamide et (+)-2-hydroxy-3-[1-(1H-imidazol-4-yl)éthyl]benzènecarboximidamide.

a) Le 2-hydroxy-3-[1-(1H-imidazol-4-yl)éthyl]-benzènecarboximidate de méthyle est préparé comme à l'exemple 2.2.a) au départ de 32 g (0,150 mole) de 2-hydroxy-3-[1-(1H-imidazol-4-yl)éthyl]-benzonitrile (préparé à l'exemple 1.2). Le résidu brut de 2-hydroxy-3-[1-(1H-imidazol-4-yl)éthyl]-benzènecarboximidate de méthyle est utilisé tel quel dans l'étape suivante.
b) On reprend le résidu obtenu en a) ci-dessus dans 150 ml d'éthanol absolu et on y ajoute 38,7 ml (0,3 mole) de S-(-)-α-méthylbenzylamine. On laisse réagir à la température ambiante pendant 15 heures, puis on évapore le solvant sous pression réduite. On purifie le résidu obtenu par chromatographie liquide préparative (silice: 1 kg; éluant: mélange 95,6:4:0,4 (v/v/v) chloroforme - méthanol - ammoniaque).
c) Le mélange de diastéréoisomères, partiellement purifiés, est ensuite chromatographié par fraction de 5 g (silice: 1 kg; éluant: mélange 94,5:5:0,5 (v/v/v) acétate d'éthyle - méthanol - ammoniaque) pour effectuer la séparation complète des diastéréoisomères. Les deux diastéréoisomères pratiquement purs sont encore chromatographiés une dernière fois dans les mêmes conditions. On obtient ainsi 18,9 g (rendement: 33%) du diastéréoisomère A, le moins polaire et donc le premier élué, et 27,3 g (rendement 47%) du diastéréoisomère B, le plus polaire et donc le dernier élué. Ces deux composés sont respectivement engagés dans l'étape de débenzylation finale.

RMN du diastéréoisomère A:

δ: 1,40 (3H,d), 1,51 (3H,d), 4,53 (1H,q), 4,99 (1H,q), 6,29 (1H,dd), 6,65 (1H,s), 6,91 (1H,dd), 7,2 à 7,5 (7H,m), 7,75 (1H,m)

RMN du diastéréoisomère B:

δ: 1,40 (3H,d), 1,52 (3H,d), 4,53 (1H,q), 4,99 (1H,q), 6,27 (1H,dd), 6,67 (1H,s), 6,89 (1H,dd), 7,2 à 7,5 (7H,m), 7,75 (1H,m)

d) (-)-2-hydroxy-3-[1-(1H-imidazol-4-yl)éthyl]-benzènecarboximidamide.
On chauffe à reflux pendant 24 heures une solution contenant 30 g du diastéréoisomère A (isolé en c) ci-dessus) dans 300 ml d'une solution aqueuse d'acide chlorhydrique 12N et 30 ml de toluène. Après élimination par filtration d'un solide résiduel, on décante la phase organique et on lave la phase aqueuse avec un peu de toluène. La phase aqueuse est ensuite alcalinisée à pH 9 au moyen d'une solution aqueuse d'hydroxyde de sodium 1 N. On extrait cette solution aqueuse avec un mélange 80:20 (v/v) acétate d'éthyle - méthanol. L'évaporation des solvants organiques d'extraction fournit un résidu solide (1$^{er}$ jet). On évapore également la phase aqueuse et on reprend le résidu obtenu par 30 ml d'une solution d'ammoniac 2,5 N dans l'alcool isopropylique. On filtre les sels insolubles et on évapore l'alcool isopropylique pour obtenir un résidu solide (2$^{ème}$ jet). Les deux jets réunis sont purifiés par deux chromatographies liquides préparatives successives effectuées dans les mêmes conditions (silice: 1 kg; éluant: mélange 78:20:2 (v/v/v) dichlorométhane - méthanol - ammoniaque). On isole 10 g de produit solide blanc que l'on recristallise dans de l'eau. On obtient 7,18 g de (-)-2-hydroxy-3-[1-(1H-imidazol-4-yl)éthyl]-benzènecarboximidamide monohydraté. Rendement: 32%.

P.F.: 125,18°C
RMN: δ: 1,40 (3H,d), 4,44 (1H,q), 6,16 (1H,t), 6,65 (1H,s), 6,90 (1H,dd), 7,41 (1H,dd), 7,44 (1H,s)
$[\alpha]_D^{25}$ = -232,36° (c=1, méthanol)

| Analyse pour $C_{12}H_{14}N_4O.H_2O$ en % : | | | |
|---|---|---|---|
| calculé: | C 58,04 | H 6,49 | N 22,57 |
| trouvé: | 57,86 | 6,54 | 22,65 |

e) (+)-2-hydroxy-3-[1-(1H-imidazol-4-yl)éthyl]-benzènecarboximidamide.

On chauffe à reflux pendant 24 heures une solution contenant 27,3 g du diastéréoisomère B (isolé en c) ci-dessus) dans 270 ml d'une solution aqueuse d'acide chlorhydrique 12N et 27 ml de toluène. On élimine la phase organique par décantation et on lave la phase aqueuse avec un peu de toluène. On évapore la phase aqueuse sous pression réduite et on reprend le résidu dans 100 ml d'éthanol. Ensuite, on neutralise la solution éthanolique avec 40 ml d'une solution d'ammoniac 2,5 N dans l'alcool isopropylique. On filtre les sels insolubles et on évapore le filtrat. On purifie le résidu obtenu par deux chromatographies liquides préparatives successives effectuées dans les mêmes conditions (silice: 800 g; éluant: mélange 78:20:2 (v/v/v) dichlorométhane - méthanol - ammoniaque). On isole 6,22 g de produit solide blanc que l'on recristallise dans de l'eau. On obtient 4,93 g de (+)-2-hydroxy-3-[1-(1H-imidazol-4-yl)éthyl]-benzènecarboximidamide monohydraté. Rendement: 24 %.

P.F.: 139,07°C

RMN: δ: 1,40 (3H,d), 4,48 (1H,q), 6,14 (1H,t), 6,63 (1H,s), 7,40 (1H,s), 7,41 (1H,dd)

$[\alpha]_D^{25}$ = + 237,44° (c=1, méthanol)

| Analyse pour $C_{12}H_{14}N_4O.H_2O$ en % : | | | |
|---|---|---|---|
| calculé: | C 58,04 | H 6,49 | N 22,57 |
| trouvé: | 58,11 | 6,53 | 22,65 |

Exemple 5. Préparation de 2-hydroxy-3-[1-(1H-imidazol-4-yl)alkyl]-benzènecarboximidamides substitués de formule I.

1. 2-hydroxy-3-(1H-imidazol-4-ylméthyl)-benzènecarboximidamide.

a) On ajoute 11,3 ml (0,0088 mole) de S-(-)-α-méthylbenzylamine à 140 ml de la solution de 2-hydroxy-3-(1H-imidazol-4-ylméthyl)-benzènecarboximidate de méthyle dans l'éthanol absolu préparée à l'exemple 2.5 a) ci-dessus. On laisse réagir pendant 15 heures à 20°C, puis on évapore le solvant sous pression réduite. On purifie le résidu obtenu par chromatographie liquide préparative (silice: 1 kg; éluant: 94,5:5:0,5 (v/v/v) acétate d'éthyle - méthanol - ammoniaque). On obtient ainsi 13,9 g de 2-hydroxy-3-(1H-imidazol-4-ylméthyl)-N-α-méthylbenzyl-benzènecarboximidamide que l'on utilise tel quel dans l'étape suivante.

b) Aux fins d'analyse, on chromatographie encore une fois 2 g du 2-hydroxy-3-(1H-imidazol-4-ylméthyl)-N-(α-méthylbenzyl-benzènecarboximidamide préparé en 1.a) ci-dessus (silice: 500 g; éluant: 92,3:7:0,7 (v/v/v) dichlorométhane - méthanol - ammoniaque).

RMN: δ: 1,52 (3H,d), 3,74 (2H,m), 5,00 (1H,q), 6,28 (1H,t), 6,64 (1H,s), 6,96 (1H,dd), 7,25 à 7,45 (6H,m), 7,51 (1H,dd), 7,80 (1H,b)

$[\alpha]_D^{25}$ = + 225° (c=1, méthanol)

c) On introduit dans un digesteur, 1 g (0,0031 mole) de 2-hydroxy-3-(1H-imidazol-4-ylméthyl)-N-α-méthylbenzyl-benzènecarboximidamide préparé en 1.a) ci-dessus et 15 ml d'ammoniac et on chauffe à 100°C pendant 40 heures. On évapore le solvant et on purifie le résidu par chromatographie liquide préparative (silice: 200 g; éluant: mélange 78:20:2 (v/v/v) dichlorométhane - méthanol - ammoniaque). On obtient, après évaporation des solvants, 0,5 g de 2-hydroxy-3-(1H-imidazol-4-ylméthyl)-benzènecarboximidamide, composé qui est identique à celui préparé à l'exemple 2.1. Rendement: 75%.

2. 2-hydroxy-3-(1H-imidazol-4-ylméthyl)-N-(1-méthyléthyl)-benzènecarboximidamide.

On introduit dans un digesteur 5 g (0,0156 mole) de 2-hydroxy-3-(1H-imidazol-4-ylméthyl)-N-α-méthylbenzyl-benzènecarboximidamide préparé en 1.a) ci-dessus et 50 ml de 2-propanamine et on chauffe à 80°C pendant 54 heures, puis à 100°C pendant 46 heures. On évapore le solvant et on purifie le résidu par chromatographie liquide préparative (silice: 600 g; éluant: mélange 89:10:1 (v/v/v) dichlorométhane - méthanol - ammoniaque). On obtient, après évaporation des solvants, 3,6 g d'un produit solide jaune pâle que l'on recristallise deux fois dans de l'acétonitrile. On obtient 1,25 g de 2-hydroxy-3-(1H-imidazol-4-ylméthyl)-N-(1-méthyléthyl)-benzènecarboximidamide. Rendement: 31%.

P.F.: 138-139°C (décomposition).
RMN: δ: 1,24 (6H,d), 3,70 (2H,s), 3,91 (1H,m), 6,20 (1H,t), 6,60 (1H,s), 6,94 (1H,d), 7,43 (1H,s), 7,48 (1H,dd), 7,98 (1H,b), 11,7 (1H,b).

| Analyse pour $C_{14}H_{18}N_4O.H_2O$ en % | | | |
|---|---|---|---|
| calculé: | C 60,85 | H 7,30 | N 20,28 |
| trouvé: | 60,62 | 7,67 | 19,88 |

## Revendications

**1.** 2-hydroxy-3-[1-(1H-imidazol-4-yl)alkyl]-benzènecarboximidamides substitués, leurs isomères optiques et leurs mélanges racémiques répondant à la formule générale

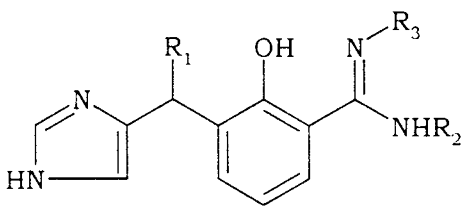

(I)

dans laquelle

R$_1$ représente un atome d'hydrogène ou un radical alkyle ayant de 1 à 4 atomes de carbone;
R$_2$ représente un atome d'hydrogène, un groupe hydroxyle, un groupe amino ou un radical alkyle ayant de 1 à 4 atomes de carbone;
R$_3$ représente un atome d'hydrogène, ou
R$_2$ et R$_3$ pris ensemble, représentent le groupe $-CH_2-CH_2-$,

ainsi que leurs sels d'addition d'acides non toxiques ophtalmologiquement acceptables.

**2.** 2-hydroxy-3-[1-(1H-imidazol-4-yl)alkyl]-benzènecarboximidamides substitué selon la revendication 1 caractérisé en ce qu'il s'agit du 2-hydroxy-3-(1H-imidazol-4-ylméthyl)-benzènecarboximidamide et ses sels d'addition d'acides non toxiques ophtalmologiquement acceptables.

**3.** 2-hydroxy-3-[1-(1H-imidazol-4-yl)alkyl]-benzénecarboximidamides substitué selon la revendication 1 caractérisé en ce qu'il s'agit du N,2-dihydroxy-3-(1H-imidazol-4-ylméthyl)-benzènecarboximidamide et ses sels d'addition d'acides non toxiques ophtalmologiquement acceptables.

**4.** 2-hydroxy-3-[1-(1H-imidazol-4-yl)alkyl]-benzènecarboximidamides substitué selon la revendication 1 caractérisé en ce qu'il s'agit du 2-hydroxy-3-(1H-imidazol-4-ylméthyl)-N-méthyl-benzènecarboximidamide et ses sels d'addition d'acides non toxiques ophtalmologiquement acceptables.

**5.** 2 -hydroxy-3-[1-(1H-imidazol-4-yl)alkyl]-benzènecarboximidamides substitué selon la revendication 1 caractérisé

en ce qu'il s'agit du 2-hydroxy-3-[1-(1H-imidazol-4-yl)éthyl]-benzènecarboximidamide et ses sels d'addition d'acides non toxiques ophtalmologiquement acceptables.

**6.** 2 -hydroxy-3-[1-(1H-imidazol-4-yl)alkyl]-benzènecarboximidamides substitué selon la revendication 1 caractérisé en ce qu'il s'agit du (+)-2-hydroxy-3-[1-(1H-imidazol-4-yl)éthyl]-benzènecarboximidamide et ses sels d'addition d'acides non toxiques ophtalmologiquement acceptables.

**7.** 2-hydroxy-3-[1-(1H-imidazol-4-yl)alkyl]-benzènecarboximidamides substitué selon la revendication 1 caractérisé en ce qu'il s'agit du (-)-2-hydroxy-3-[1-(1H-imidazol-4-yl)éthyl]-benzènecarboximidamide et ses sels d'addition d'acides non toxiques ophtalmologiquement acceptables.

**8.** 2-hydroxy-3-[1-(1H-imidazol-4-yl)alkyl]-benzènecarboximidamides substitué selon la revendication 1 caractérisé en ce qu'il s'agit du 2-(4,5-dihydro-1H-imidazol-2-yl)-6-(1H-imidazol-4-ylméthyl)-phénol et ses sels d'addition d'acides non toxiques ophtalmologiquement acceptables.

**9.** 2-hydroxy-3-[1-(1H-imidazol-4-yl)alkyl]-benzènecarboximidamides substitué selon la revendication 1 caractérisé en ce qu'il s'agit du 2-hydroxy-3-(1H-imidazol-4-ylméthyl)-N-(1-methyléthyl)-benzènecarboximidamide et ses sels d'addition non toxiques ophtalmologiquement acceptables.

**10.** 2-hydroxy-3-[1-(1H-imidazol-4-yl)alkyl]-benzènecarboximidamides substitué selon la revendication 1 caractérisé en ce qu'il s'agit de l'hydrazide de l'acide 2-hydroxy-3-(1H-imidazol-4-ylméthyl)-benzènecarboximidique et ses sels d'addition d'acides non toxiques ophtalmologiquement acceptables.

**11.** Procédé de préparation de 2-hydroxy-3-[1-(1H-imidazol-4-yl)alkyl]-benzènecarboximidamides substitués et de leurs sels tels que définis dans la revendication 1, caractérisé en ce que:

a)

(1) on fait réagir un 2-hydroxy-3-[1-(1H-imidazol-4yl)alkyl]-benzonitrile de formule

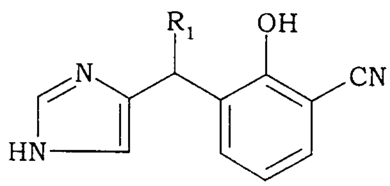

(II)

dans laquelle $R_1$ à la signification donnée à la revendication 1, avec un alcanol ayant de 1 à 4 atomes de carbone en présence d'acide chlorhydrique gazeux;
(2) on fait réagir ensuite le 2-hydroxy-3-[1-(1H-imidazol-4-yl)alkyl]-benzènecarboximidate d'alkyle ainsi obtenu de formule

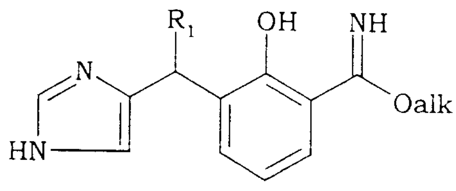

(III)

dans laquelle $R_1$ a la signification donnée à la revendication 1 et alk représente un radical alkyle ayant de 1 à 4 atomes de carbone, avec un des énantiomères de l'α-méthylbenzylamine; et
(3) on fait réagir finalement le 2-hydroxy-3-[1-(1H-imidazol-4-yl)alkyl]-N-α-méthylbenzyl-benzènecarboximidamide ainsi obtenu de formule

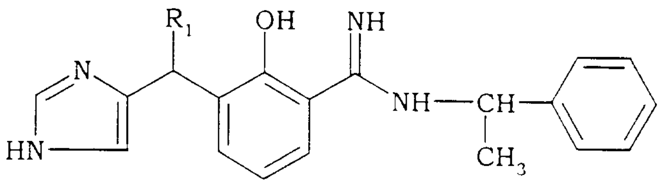

(IV)

dans laquelle $R_1$ a la signification donnée à la revendication 1, avec un composé azoté de formule $R_2NH_2$ (V) dans laquelle $R_2$ représente un atome d'hydrogène, un groupe hydroxyle, un groupe amino ou un radical alkyle ayant de 1 à 4 atomes de carbone lorsque $R_3$ est de l'hydrogène ou avec l'éthylènediamine (VI) lorsque $R_2$ et $R_3$, pris ensemble, représentent le groupe $-CH_2-CH_2-$, et lorsqu'on désire obtenir un composé de formule I sous forme d'un isomère optique, on sépare au préalable les dérivés N-α-méthylbenzylés diastéréoisomères de formule IV, dans laquelle $R_1$ représente un radical alkyle ayant de 1 à 4 atomes de carbone; ou

b) on fait réagir un 2-hydroxy-3-[1-(1H-imidazol-4-yl)alkyl]-benzènecarboximidate d'alkyle de formule III obtenu de la manière décrite au point a) ci-dessus avec un composé azoté de formule $R_2NH_2$ (V) dans laquelle $R_2$ représente un atome d'hydrogène, un groupe hydroxyle, un groupe amino ou un radical alkyle ayant de 1 à 4 atomes de carbone lorsque $R_3$ est de l'hydrogène ou avec l'éthylènediamine (VI) lorsque $R_2$ et $R_3$, pris ensemble, représentent le groupe $-CH_2-CH_2-$; ou

c) pour préparer les 2-hydroxy-3-[1-(1H-imidazol-4-yl)alkyl]-benzènecarboximidamides répondant à la formule générale I dans laquelle $R_2$ représente un groupe hydroxyle et $R_3$ représente un atome d'hydrogène, on fait réagir l'hydroxylamine avec un 2-hydroxy-3-[1-(1H-imidazol-4-yl)alkyl]-benzonitrile de formule

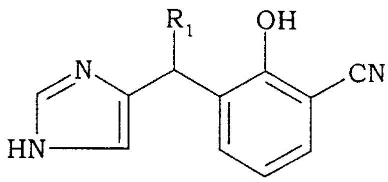

(II)

dans laquelle $R_1$ a la signification donnée à la revendication 1: ou

d) pour préparer les 2-hxdroxy-3-[1-(1H-imidazol-4-yl)alkyl]-benzènecarboximidamide répondant à la formule générale I dans laquelle $R_1$ représente un radical alkyle ayant de 1 à 4 atomes de carbone, et $R_2$ et $R_3$ représentent tout deux un atome d'hydrogène, sous forme de leurs isomères optiques, on sépare les 2-hydroxy-3-[1-(1H-imidazol-4-yl)alkyl]-N-α-méthylbenzyl-benzènecarboximidamides diastéréoisomères de formule IV obtenus de la manière décrite au point a) ci-dessus et on élimine ensuite le groupe α-méthylbenzyle de chaque diastéréoisomère ainsi séparé, par hydrolyse au moyen d'acide chlorhydrique concentré à une température comprise entre 80°C et 110°C; et

e) en ce que, éventuellement on convertit les 2-hydroxy-3-[1-(1H-imidazol-4-yl)alkyl]-benzènecarboximidamides de formule I ainsi obtenus en leurs sels d'addition d'acides non toxiques ophtalmologiquement acceptables.

**12.** 2-hydroxy-3-[1-(1H-imidazol-4-yl)alkyl]-benzènecarboximidamides selon l'une quelconque des revendications 1 à 10 ou un de leurs sels d'addition d'acides non toxiques ophtalmologiquement acceptables pour utilisation dans la prévention et le traitement du glaucome.

**13.** Compositions ophtalmiques pour diminuer la pression intraoculaire comprenant une quantité efficace d'un ou plusieurs 2-hydroxy-3-[1-(1H-imidazol-4-yl)alkyl]-benzènecarboximidamides selon l'une quelconque des revendications 1 à 10 ou d'un de leurs sels d'addition d'acides non toxiques ophtalmologiquement acceptables, en association avec un ou plusieurs excipients ophtalmologiques non toxiques, solides ou liquides.

**Claims**

1. Substituted 2-hydroxy-3-[1-(1H-imidazol-4-yl)alkyl]-benzenecarboximidamides, their optical isomers and their racemic mixtures corresponding to the general formula

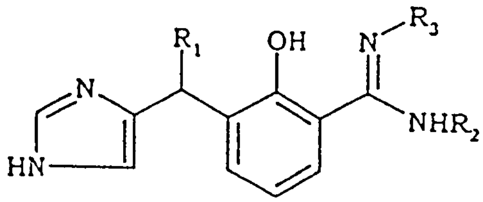

(I)

in which

| | |
|---|---|
| $R_1$ | represents a hydrogen atom or an alkyl radical having 1 to 4 carbon atoms; |
| $R_2$ | represents a hydrogen atom, a hydroxyl group, an amino group or an alkyl radical having 1 to 4 carbon atoms; |
| $R_3$ | represents a hydrogen atom, or |
| $R_2$ and $R_3$ | taken together represent the $-CH_2-CH_2-$ group, |

as well as their addition salts of ophthalmologically acceptable non-toxic acids.

2. Substituted 2-hydroxy-3-[1-(1H-imidazol-4-yl)alkyl]-benzenecarboximidamides according to claim 1, characterized in that it concerns 2-hydroxy-3-(1H-imidazol-4-ylmethyl)-benzenecarboximidamide and its addition salts of ophthalmologically acceptable non-toxic acids.

3. Substituted 2-hydroxy-3-[1-(1H-imidazol-4-yl)alkyl]-benzenecarboximidamides according to claim 1, characterized in that it concerns N,2-dihydroxy-3-(1H-imidazol-4-ylmethyl)-benzenecarboximidamide and its addition salts of ophthalmologically acceptable non-toxic acids.

4. Substituted 2-hydroxy-3-[1-(1H-imidazol-4-yl)alkyl]-benzenecarboximidamides according to claim 1, characterized in that it concerns 2-hydroxy-3-(1H-imidazol-4-ylmethyl)-N-methyl-benzenecarboximidamide and its addition salts of ophthalmologically acceptable non-toxic acids.

5. Substituted 2-hydroxy-3-[1-(1H-imidazol-4-yl)alkyl]-benzenecarboximidamides according to claim 1, characterized in that it concerns 2-hydroxy-3-[1-(1H-imidazol-4-yl)ethyl]-benzenecarboximidamide and its addition salts of ophthalmologically acceptable non-toxic acids.

6. Substituted 2-hydroxy-3-[1-(1H-imidazol-4-yl)alkyl]-benzenecarboximidamides according to claim 1, characterized in that it concerns (+)-2-hydroxy-3-[1-(1H-imidazol-4-yl)ethyl]-benzenecarboximidamide and its addition salts of ophthalmologically acceptable non-toxic acids.

7. Substituted 2-hydroxy-3-[1-(1H-imidazol-4-yl)alkyl]-benzenecarboximidamides according to claim 1, characterized in that it concerns (-)-2-hydroxy-3-[1-(1H-imidazol-4-yl)ethyl]-benzenecarboximidamide and its addition salts of ophthalmologically acceptable non-toxic acids.

8. Substituted 2-hydroxy-3-[1-(1H-imidazol-4-yl)alkyl]-benzenecarboximidamides according to claim 1, characterized in that it concerns 2-(4,5-dihydro-1H-imidazol-2-yl)-6-(1H-imidazol-4-ylmethyl)-phenol and its addition salts of ophthalmologically acceptable non-toxic acids.

9. Substituted 2-hydroxy-3-[1-(1H-imidazol-4-yl)alkyl]-benzenecarboximidamides according to claim 1, characterized in that it concerns 2-hydroxy-3-(1H-imidazol-4-ylmethyl)-N-(1-methylethyl)-benzenecarboximidamide and its addition salts of ophthalmologically acceptable non-toxic acids.

**10.** Substituted 2-hydroxy-3-[1-(1H-imidazol-4-yl)alkyl]-benzenecarboximidamides according to claim 1, characterized in that it concerns the hydrazide of 2-hydroxy-3-(1H-imidazol-4-ylmethyl]-benzenecarboximidic acid and its addition salts of ophthalmologically acceptable non-toxic acids.

**11.** Method for preparing substituted 2-hydroxy-3-[1-(1H-imidazol-4-yl)alkyl]-benzenecarboximidamides and their salts as defined in claim 1, characterized in that:

a)

(1) a 2-hydroxy-3-[1-(1H-imidazol-4-yl)alkyl]-benzonitrile of formula

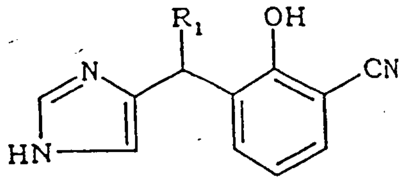

(II)

in which $R_1$ has the meaning given in claim 1, is reacted with an alkanol having 1 to 4 carbon atoms in the presence of gaseous hydrochloric acid;

(2) the alkyl 2-hydroxy-3-[1-(1H-imidazol-4-yl)alkyl]-benzenecarboximidate so obtained, of formula

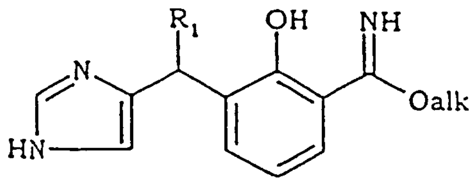

(III)

in which $R_1$ has the meaning given in claim 1 and alk represents an alkyl radical having 1 to 4 carbon atoms, is then reacted with one of the enantiomers of $\alpha$-methylbenzylamine; and

(3) the 2-hydroxy-3-[1-(1H-imidazol-4-yl)alkyl]-N-$\alpha$-methylbenzyl-benzenecarboximidamide thus obtained of formula

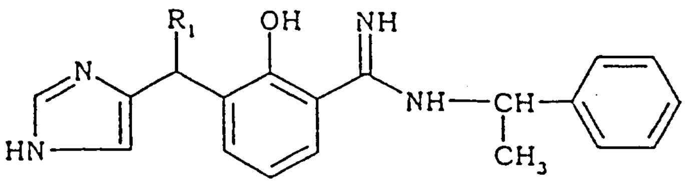

(IV)

in which $R_1$ has the meaning given in claim 1, is finally reacted with a nitrogen-containing compound of the formula $R_2NH_2$ (V) in which $R_2$ represents a hydrogen atom, a hydroxyl group, an amino group or an alkyl radical having 1 to 4 carbon atoms when $R_3$ is hydrogen or with ethylenediamine (VI) when $R_2$ and $R_3$ taken together represent the $-CH_2-CH_2-$ group, and when it is desired to obtain a compound of formula 1 in the form of an optical isomer, the diastereoisomeric N-$\alpha$-methylbenzylated derivatives of formula IV, in which $R_1$ represents an alkyl radical having 1 to 4 carbon atoms, are first of all separated; or

b) an alkyl 2-hydroxy-3-[1-(1H-imidazol-4-yl)alkyl]-benzenecarboximidate of formula III obtained in the manner described in item a) above is reacted with a nitrogen-containing compound of the formula $R_2NH_2$ (V) in which $R_2$ represents a hydrogen atom, a hydroxyl group, an amino group or an alkyl radical having 1 to 4 carbon atoms when $R_3$ is hydrogen or with ethylenediamine (VI) when $R_2$ and $R_3$ taken together represent the $-CH_2-CH_2-$ group; or

c) in order to prepare the 2-hydroxy-3-[1-(1H-imidazol-4-yl)alkyl]-benzenecarboximidamides corresponding to the general formula 1 in which $R_2$ represents a hydroxyl group and $R_3$ represents a hydrogen atom, hydroxylamine is reacted with a 2-hydroxy-3-[1-(1H-imidazol-4-yl)alkyl)]-benzonitrile of formula

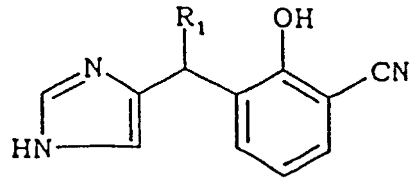

(II)

in which $R_1$ has the meaning given in claim 1; or

d) in order to prepare the 2-hydroxy-3-[1-(1H-imidazol-4-yl)alkyl]-benzenecarboximidamides corresponding to the general formula 1 in which $R_1$ represents an alkyl radical having 1 to 4 carbon atoms, and $R_2$ and $R_3$ both represent a hydrogen atom, in the form of their optical isomers, the diastereoisomeric 2-hydroxy-3-[1-(1H-imidazol-4-yl)alkyl]-N-α-methylbenzyl-benzenecarboximidamides of formula IV obtained in the manner described in item a) above are separated and the α-methylbenzyl group is then eliminated from each diastereoisomer thus separated, by hydrolysis by means of concentrated hydrochloric acid at a temperature of between 80°C and 110°C; and

e) in that the 2-hydroxy-3-[1-(1H-imidazol-4-yl)alkyl]-benzenecarboximidamides of formula I thus obtained are optionally converted into their addition salts of ophthalmologically acceptable non-toxic acids.

**12.** 2-hydroxy-3-[1-(1H-imidazol-4-yl)alkyl]-benzenecarboximidamides according to any one of claims 1 to 10 or one of their addition salts of ophthalmologically acceptable non-toxic acids for use in the prevention and treatment of glaucoma.

**13.** Ophthalmic compositions for reducing the intra-ocular pressure containing an effective quantity of one or more 2-hydroxy-3-[1-(1H-imidazol-4-yl)alkyl]-benzenecarboximidamides according to any one of claims 1 to 10 or of one of their addition salts of ophthalmologically acceptable non-toxic acids, in association with one or more solid or liquid ophthalmologically non-toxic excipients.

## Patentansprüche

**1.** Substituierte 2-Hydroxy-3-[1-(1H-imidazol-4-yl)alkyl]-benzencarboximidamide, deren optische Isomere und deren racemische Gemische, die der allgemeinen Formel

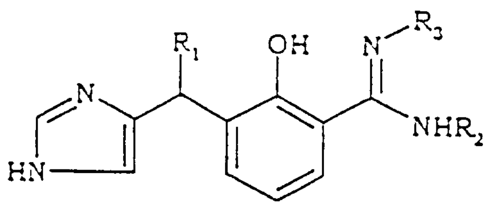

(I)

entsprechen, in der

| | |
|---|---|
| $R_1$ | ein Wasserstoffatom oder einen Alkylrest mit 1 bis 4 Kohlenstoffatomen darstellt; |
| $R_2$ | ein Wasserstoffatom, ein Hydroxygruppe, eine Aminogruppe oder einen Alkylrest mit 1 bis 4 Kohlenstoffatomen darstellt; |
| $R_3$ | ein Wasserstoffatom darstellt, oder |
| $R_2$ und $R_3$ | zusammen genommen die Gruppe $-CH_2-CH_2-$ darstellen, |

sowie deren Additionssalze mit Säuren, die ophthalmologisch annehmbar und nicht toxisch sind.

**2.** Substituiertes 2-Hydroxy-3-[1-(1H-imidazol-4-yl)alkyl]-benzencarboximidamid gemäß Anspruch 1, dadurch gekennzeichnet, dass es sich um 2-Hydroxy-3-(1H-imidazol-4-ylmethyl)-benzencarboximidamid und seine Additionssalze mit Säuren, die ophthalmologisch annehmbar und nicht toxisch sind, handelt.

**3.** Substituiertes 2-Hydroxy-3-[1-(1H-imidazol-4-yl)alkyl]-benzencarboximidamid gemäß Anspruch 1, dadurch gekennzeichnet, dass es sich um N,2-Dihydroxy-3-(1H-imidazol-4-ylmethyl)-benzencarboximidamid und seine Additionssalze mit Säuren, die ophthalmologisch annehmbar und nicht toxisch sind, handelt.

**4.** Substituiertes 2-Hydroxy-3-[1-(1H-imidazol-4-yl)alkyl]-benzencarboximidamid gemäß Anspruch 1, dadurch gekennzeichnet, dass es sich um 2-Hydroxy-3-(1H-imidazol-4-ylmethyl)-N-methyl-benzencarboximidamid und seine Additionssalze mit Säuren, die ophthalmologisch annehmbar und nicht toxisch sind, handelt.

**5.** Substituiertes 2-Hydroxy-3-[1-(1H-imidazol-4-yl)alkyl]-benzencarboximidamid gemäß Anspruch 1, dadurch gekennzeichnet, dass es sich um 2-Hydroxy-3-[1-(1H-imidazol-4-yl)ethyl]-benzencarboximidamid und seine Additionssalze mit Säuren, die ophthalmologisch annehmbar und nicht toxisch sind, handelt

**6.** Substituiertes 2-Hydroxy-3-[1-(1H-imidazol-4-yl)alkyl]-benzencarboximidamid gemäß Anspruch 1, dadurch gekennzeichnet, dass es sich um (+)-2-Hydroxy3-[1-(1H-imidazol-4-yl)ethyl]-benzencarboximidamid und seine Additionssalze mit Säuren, die ophthalmologisch annehmbar und nicht toxisch sind, handelt.

**7.** Substituiertes 2-Hydroxy-3-[1-(1H-imidazol-4-yl)alkyl]-benzencarboximidamid gemäß Anspruch 1, dadurch gekennzeichnet, dass es sich um (-)-2-Hydroxy-3-[1-(1H-imidazol-4-yl)ethyl]-benzencarboximidamid und seine Additionssalze mit Säuren, die ophthalmologisch annehmbar und nicht toxisch sind, handelt

**8.** Substituiertes 2-Hydroxy-3-[1-(1H-imidazol-4-yl)alkyl]-benzencarboximidamid gemäß Anspruch 1, dadurch gekennzeichnet, dass es sich um 2-(4,5-Dihydro-1H-imidazol-2-yl)-6-(1H-imidazol-4-ylmethyl)-phenol und seine Additionssalze mit Säuren, die ophthalmologisch annehmbar und nicht toxisch sind, handelt.

**9.** Substituiertes 2-Hydroxy-3-[1-(1H-imidazol-4-yl)alkyl]-benzencarboximidamid gemäß Anspruch 1, dadurch gekennzeichnet, dass es sich um 2-Hydroxy-3-(1H-imidazol-4-ylmethyl)-N-(1-methylethyl)-benzencarboximidamid und seine Additionssalze mit Säuren, die ophthalmologisch annehmbar und nicht toxisch sind, handelt.

**10.** Substituiertes 2-Hydroxy-3-[1-(1H-imidazol-4-yl)alkyl]-benzencarboximidamid gemäß Anspruch 1, dadurch gekennzeichnet, dass es sich um das Hydrazid der 2-Hydroxy-3-(1H-imidazol-4-ylmethyl)-benzencarboximidsäure und seine Additionssalze mit Säuren, die ophthalmologisch annehmbar und nicht toxisch sind, handelt.

**11.** Verfahren zur Herstellung von substituierten 2-Hydroxy-3-[1-(1H-imidazol4-yl)alkyl]-benzencarboximidamiden und deren Salzen, wie in Anspruch 1 definiert, dadurch gekennzeichnet, dass:

a)

(1) man ein 2-Hydroxy-3-[1-(1H-imidazol-4-yl)alkyl]-benzonitril der Formel

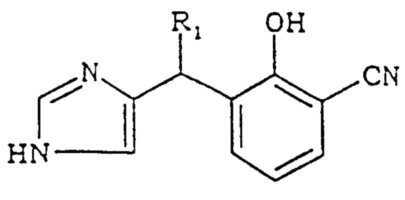

(II),

in der $R_1$ die in Anspruch 1 angegebene Bedeutung hat, mit einem Alkanol mit 1 bis 4 Kohlenstoffatomen in Gegenwart von gasförmiger Chlorwasserstoffsäure umsetzt;
(2) man dann das so erhaltene Alkyl-2-hydroxy-3-[1-(1H-imidazol-4-yl)-alkyl]-benzencarboximidat der Formel

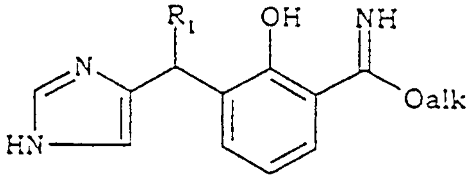

(III),

in der $R_1$ die in Anspruch 1 angegebene Bedeutung hat und alk einen Alkylrest mit 1 bis 4 Kohlenstoffatomen darstellt, mit einem der Enantiomere des α-Methylbenzylamins umsetzt; und

(3) man schließlich das so erhaltene 2-Hydroxy-3-[1-(1H-imidazol-4-yl)alkyl]-N-α-methylbenzyl-benzencarboximidamid der Formel

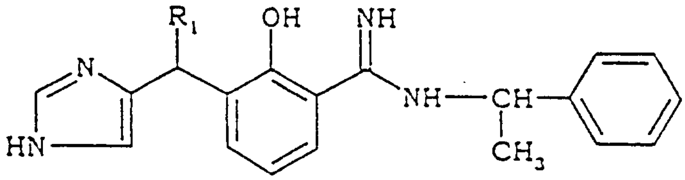

(IV),

in der $R_1$ die in Anspruch 1 angegebene Bedeutung hat, mit einer stickstoffhaltigen Verbindung der Formel $R_2NH_2$ (V), in der $R_2$ ein Wasserstoffatom, eine Hydroxygruppe, eine Aminogruppe oder einen Alkylrest mit 1 bis 4 Kohlentoffatomen darstellt, wenn $R_3$ Wasserstoff ist, oder mit Ethylendiamin (VI), wenn $R_2$ und $R_3$ zusammen genommen die Gruppe $-CH_2-CH_2-$ darstellen, umsetzt, und wenn man eine Verbindung der Formel I in Form eines optischen Isomers zu erhalten wünscht, man vorher die diastereomeren N-α-Methylbenzylderivate der Formel IV, in der $R_1$ einen Alkylrest mit 1 bis 4 Kohlenstoffatomen darstellt, trennt; oder

(b) man ein Alkyl-2-hydroxy-3-[1-(1H-imidazol-4-yl)alkyl]-benzencarboximidat der Formel III, das auf die oben unter Punkt a) beschriebene Weise erhalten wurde, mit einer stickstoffhaltigen Verbindung der Formel $R_2NH_2$ (V), in der $R_2$ ein Wasserstoffatom, eine Hydroxygruppe, eine Aminogruppe oder einen Alkylrest mit 1 bis 4 Kohlenstoffatomen darstellt, wenn $R_3$ Wasserstoff ist, oder mit Ethylendiamin (VI), wenn $R_2$ und $R_3$ zusammen genommen die Gruppe $-CH_2-CH_2-$ darstellen, umsetzt; oder c) um die 2-Hydroxy-3-[1-(1H-imidazol-4-yl)alkyl]-benzencarboximidamide die der allgemeinen Formel I entsprechen, in der $R_2$ eine Hydroxygruppe darstellt und $R_3$ ein Wasserstoffatom darstellt, herzustellen, man Hydroxylamin mit einem 2-Hydroxy-3-[1-(1H-imidazol-4-yl)alkyl]-benzonitril der Formel

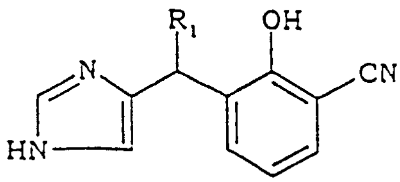

(II),

in der $R_1$ die in Anspruch 1 angegebene Bedeutung hat, umsetzt; oder

d) um die 2-Hydroxy-3-[1-(1H-imidazol-4-yl)alkyl]-benzencarboximidamide die der allgemeinen Formel I entsprechen, in der $R_1$ einen Alkylrest mit 1 bis 4 Kohlenstoffatomen darstellt und $R_2$ und $R_3$ beide ein Wasserstoffatom darstellen, in Form ihrer optischen Isomere herzustellen, man die 2-Hydroxy-3-[1-(1H-imidazol-4-yl)alkyl]-N-α-methylbenzyl-benzencarboximidamid-Diastereomere der Formel IV, die auf die oben unter Punkt a) beschriebene Weise erhalten wurden, trennt und man dann die α-Methylbenzylgruppe von jedem so abgetrennten Diastereomer durch Hydrolyse mittels konzentrierter Chlorwasserstoffsäure bei einer Temperatur zwischen 80 °C und 110 °C eliminiert; und

e) dadurch, dass man gegebenenfalls die so erhaltenen 2-Hydroxy-3-[1-(1H-imidazol-4-yl)alkyl]-benzencarboximidamide der Formel I in ihre Additionssalze mit Säuren, die ophthalmologisch annehmbar und nicht toxisch sind umwandelt.

**12.** 2-Hydroxy-3-[1-(1H-imidazol-4-yl)alkyl]-benzencarboximidamide gemäß einem der Ansprüche 1 bis 10 oder eines ihrer Additionssalze mit Säuren, die ophthalmologisch annehmbar und nicht toxisch sind, zur Verwendung bei der Vorbeugung und Behandlung des Glaukoms.

**13.** Ophthalmische Zusammensetzungen zur Verminderung des intraokularen Drucks, die eine wirksame Menge eines oderer mehrerer 2-Hydroxy-3-[1-(1H-imidazol-4-yl)alkyl]-benzencarboximidamide gemäß einem der Ansprüche 1 bis 10 oder eines ihrer Additionssalze mit Säuren, die ophthalmologisch annehmbar und nicht toxisch sind, in Verbindung mit einem oder mehreren festen oder flüssigen, nicht toxischen ophthalmologischen Exzipienten umfassen.